# EUROPEAN PATENT APPLICATION

(11) **EP 3 617 305 A1**
(43) Date of publication of application: **04.03.2020**
(21) Application number: 17868484.1
(22) Date of filing: 20.09.2017
(51) Int. Cl.: C12M 1/34, C12Q 1/68

(54) **GENETIC TESTING CHIP, TESTING METHOD AND MANUFACTURING METHOD THEREFOR, AND MICROFLUIDIC CHIP SYSTEM**

(30) Priority: 27.04.2017 CN 201710289072
(71) Applicant: BOE Technology Group Co., Ltd., Beijing 100015 (CN); Beijing BOE Optoelectronics Technology Co., Ltd., Beijing 100176 (CN)
(72) Inventor: GU, Le, Beijing 100176 (CN)
(74) Representative: Brötz, Helmut
(86) International application number: PCT/CN2017/102514
(87) International publication number: WO 2018/196265

(57) **Abstract**

Disclosed are a gene detection chip, in which a gene detection channel is formed by an injection port, a microchannel, a reaction cell, and an exit port. The surface of the reaction cell has an aptamer, and the aptamer is modified with a fluorescent label. A detection method and a manufacturing method of the gene detection chip and a microfluidic control chip system are also disclosed. Upon a gene detection being performed, the fluorescent light of the fluorescent label is firstly quenched, and then a sample solution is introduced into the reaction cell through the injection port. If the sample solution has target gene, the target gene will hybridize and combine with the aptamer on the surface of the reaction cell, so as to recover the fluorescent light; if the sample solution does not have the target gene, the fluorescent light stays in the quenched state. The gene detection chip does not need fluorescently labeling the target gene, which not only simplifies the process of gene detection, but also prevents the interference of the fluorescent signal of the non-hybridized target gene in the gene detection result.

## Description

The present application claims priority of China Patent application No. 201710289072.6 titled "GENE DETECTION CHIP, DETECTION METHOD THEREOF, AND MICROFLUIDIC CONTROL CHIP SYSTEM" filed on April 27, 2017, the content of which is incorporated in its entirety as portion of the present application by reference herein.

### TECHNICAL FIELD

The present application relates to a technical field of gene detection, in particular to a gene detection chip, a detection method thereof, a manufacturing method thereof, and a microfluidic control chip system.

### BACKGROUND

In the existing technologies, the main methods of gene detection include direct sequencing method, fluorescent polymerase chain reaction method, and gene detection chip method. Among them, the gene detection chip has been widely favored in gene detection because of its small size, celerity and simplicity and simultaneous detection of multiple genes. The sequencing principle of the gene detection chip is hybridization sequencing, which performs the detection by hybridizing the gene target sequence in the sample and the gene chip to which the gene probe is immobilized. However, gene detection chip requires the labeling of the target gene, and the technique is complicated. Moreover, the fluorescent light signal of the non-hybridized gene molecule will also interfere with the detection result and influence it.

Therefore, how to simplify the gene detection technology and improve the effect of the fluorescent signal of the non-hybridized gene in the gene detection result on the hybrid gene is a technical problem to be solved urgently by those skilled in the art.

### SUMMARY

The embodiments of the present disclosure provide a gene detection chip including: an upper substrate and a lower substrate which are disposed opposite to each other;
the upper substrate is provided with at least one pair of ports including an injection port and an exit port, the injection port and the exit port are respectively located at two opposite ends of the upper substrate;
a surface of the lower substrate facing the upper substrate is provided with at least one reaction cell and at least one microchannel, the reaction cell is respectively connected with the injection port and the exit port through the microchannel, wherein a surface of the reaction cell has an aptamer, the aptamer is modified with a fluorescent label.

In the abovementioned gene detection chip provided by the embodiments of the present disclosure, further including: a first plastic hose connected to the injection port and a second plastic hose connected to the exit port;
the first plastic hose is configured to introduce a solution through the injection port;
the second plastic hose is configured to discharge the solution through the exit port.

In the abovementioned gene detection chip provided by the embodiments of the present disclosure, a shape of the reaction cell includes a circular shape.

In the abovementioned gene detection chip provided by the embodiments of the present disclosure, a material of the lower substrate includes glass.

In the abovementioned gene detection chip provided by the embodiments of the present disclosure, the upper substrate is a transparent substrate.

Embodiments of the present disclosure further provide a microfluidic control chip system, including: the gene detection chip according to any one of claims 1-5 and a power module;
the power module is configured to supply power for introducing the solution into the gene detection chip or discharging the solution from the gene detection chip.

In the abovementioned microfluidic control chip system provided by the embodiments of the present disclosure, the power module is a syringe pump.

Embodiments of the present disclosure provide a detection method of the abovementioned gene detection, including:
introducing a graphene oxide solution into the reaction cell to completely quench fluorescent light of the fluorescent label;
after completely quenching fluorescent light of the fluorescent label, introducing a sample solution to be detected into the reaction cell through the injection port; and
determining whether the aptamer recovers fluorescent light after the sample solution undergoes a hybridization reaction with the aptamer on the surface of the reaction cell, and if so, the sample solution has a target gene, otherwise the sample solution does not have the target gene.

In the abovementioned detection method provided by the embodiments of the present disclosure, before determining whether the aptamer recovers fluorescent light after the sample solution undergoes a hybridization reaction with the aptamer on the surface of the reaction cell, the detection method further includes:
cleaning the reaction cell.

In the abovementioned detection method provided by the embodiments of the present disclosure, cleaning of the reaction cell specifically includes:
introducing a cleaning liquid into the reaction cell through the injection port, and discharging the cleaning liquid from the exit port.

Embodiments of the present disclosure provide a manufacturing method of a gene detection chip, including:
forming at least one pair of ports including an injection port and an exit port on an upper substrate, wherein the injection port and the exit port are respectively located at two opposite ends of the upper substrate;
forming at least one reaction cell and at least one microchannel on a lower substrate, and bonding an aptamer modified with a fluorescent label on an inner surface of the reaction cell; and
adhering the upper substrate and the lower substrate to make the upper substrate cover the reaction cell and the microchannel on the lower substrate, wherein the reaction cell is respectively connected with the injection port and the exit port through the microchannel.

In the abovementioned manufacturing method provided by the embodiments of the present disclosure, bonding the aptamer modified with the fluorescent label on the inner surface of the reaction cell includes:
performing a silanization treatment on the inner surface of the reaction cell, wherein a material of the lower substrate is glass; and
using an array machine to bond the aptamer modified with the fluorescent label on the inner surface of the reaction cell.

In the abovementioned manufacturing method provided by the embodiments of the present disclosure, after bonding the aptamer modified with the fluorescent label on the inner surface of the reaction cell, the manufacturing method further includes:
introducing a graphene oxide solution into the reaction cell to completely quench fluorescent light of the fluorescent label; and
cleaning the reaction cell.

Embodiments of the present disclosure provide a gene detection chip, including: an upper substrate and a lower substrate which are disposed opposite to each other;
the upper substrate is provided with at least one pair of ports including an injection port and an exit port, the injection port and the exit port are respectively located at two opposite ends of the upper substrate;
a surface of the lower substrate facing the upper substrate is provided with at least one reaction cell and at least one microchannel, the reaction cell is respectively connected with the injection port and the exit port through the microchannel, wherein a surface of the reaction cell has a complex of an aptamer and a graphene oxide, the aptamer is modified with a fluorescent label, and the fluorescent light of the fluorescent label being in a quenched state.

### BRIEF DESCRIPTION OF THE DRAWINGS

Fig. 1 is a schematic structural diagram of a gene detection chip provided by an embodiment of the present disclosure;
Fig. 2 is a flow diagram of a gene detection method provided by an embodiment of the present disclosure;
Fig. 3 is a process diagram of a gene detection process provided by an embodiment of the present disclosure; and
Fig. 4 is a flow diagram of a manufacturing method of a gene detection chip provided by an embodiment of the present disclosure.

### DETAILED DESCRIPTION

Hereinafter, embodiments of the gene detection chip, detection method thereof, manufacturing method thereof, and the microfluidic control chip system provided by the embodiments of the present disclosure are described in detail with reference to the accompanying drawings.

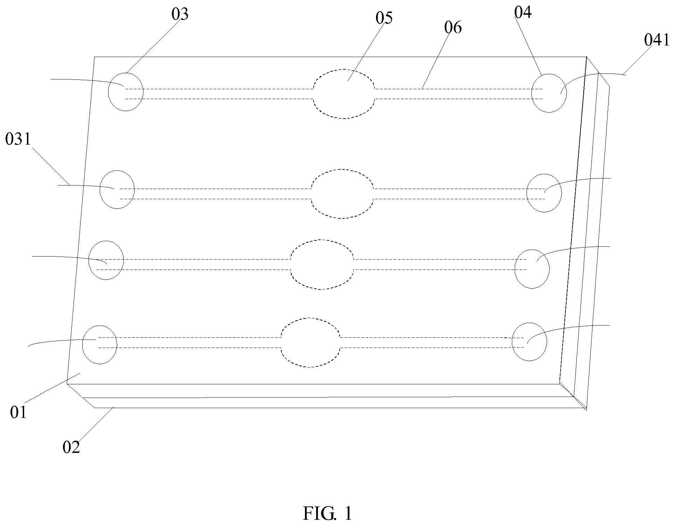

An embodiment of the present disclosure provides a gene detection chip. As illustrated by Fig. 1, the gene detection chip can include: an upper substrate 01 and a lower substrate 02 which are disposed opposite to each other; the upper substrate 01 is provided with at least one pair of ports including an injection port 03, and an exit port 04; the injection port 03 and the exit port 04 are respectively located at two opposite ends of the upper substrate 01; a surface of the lower substrate 02 facing the upper substrate 01 is provided with at least one reaction cell 05 and at least one microchannel 06; and the reaction cell 05 is respectively connected with the injection port 03 and the exit port 04 through the microchannel 06; a surface of the reaction cell 05 has an aptamer, and the aptamer is modified with a fluorescent label.

In the abovementioned gene detection chip provided by the embodiment of the present disclosure, a gene detection channel is formed by an injection port, a microchannel, a reaction cell, and an exit port. Since the surface of the reaction cell has an aptamer, and the aptamer is modified with a fluorescent label, upon a gene detection being performed, firstly quenching the fluorescent light of the fluorescent label, and then introducing a sample solution into the reaction cell through the injection port; if the sample solution has target gene, the target gene will hybridize and combine with the aptamer on the surface of the reaction cell, so as to recover the fluorescent light, if the sample solution does not have the target gene, the fluorescent light stays in the quenched state. Therefore, the gene detection chip can achieve detecting the target gene without fluorescently labeling the target gene, which not only simplifies the process of gene detection, but also prevents the influence of the fluorescent signal of the non-hybridized target gene on the hybrid gene in the gene detection result. At the same time, the gene detection chip provided by the present disclosure has a simple structure, convenient operation, high specificity of aptamer, and accurate detection result.

In addition, the gene detection chip of the present disclosure can set a plurality of gene detection channels, so as to detect multiple different genes simultaneously, thereby having high detection efficiency.

For example, in the abovementioned gene detection chip provided by the embodiment of the present disclosure, the fluorescent light of the fluorescent label can be quenched by graphene oxide. Graphene oxide is a derivative of graphene, which incorporates a hydroxyl group and a carboxyl group on the basis of graphene; compared with graphene, the graphene oxide has stronger hydrophilicity and biocompatibility. As a quencher, the graphene oxide can quench the fluorescent light of most organic dyes and quantum dots. Therefore, upon the graphene oxide solution passing through the reaction cell, a complex of the aptamer and the graphene oxide is formed on the surface of the reaction cell; in the complex of the aptamer and the graphene oxide, the graphene oxide can adsorb single-stranded nucleic acid aptamer, and then quench the fluorescent light of the label. In this way, upon the sample solution having target gene, the aptamer is combined with the complementary DNA in the target gene to form a double-stranded DNA molecule; as the steric hindrance becomes larger, the adsorption ability of the graphene oxide is weakened, and the fluorescent light recovers.

In specific implementation, in the abovementioned gene detection chip provided by the embodiment of the present disclosure, as illustrated by Fig. 1, the gene detection chip may further include: a first plastic hose 031 connected with the injection port 03 and a second plastic hose 041 connected with the exit port 04; the first plastic hose 031 is configured to introduce a solution through the injection port 03; and the second plastic hose 041 is configured to discharge the solution through the exit port 04. For example, the gene detection chip can also introduce liquid into the gene detection chip and derive the liquid in the gene chip through plastic hoses respectively connected to the injection port and the exit port, thereby facilitating the introduction and export of the solution. In addition, the genetic detection channel constituted by the injection port, microchannel, reaction cell, and exit port can be cleaned through the plastic hoses, that is, after the hybridization reaction is performed, and before determining whether there is target gene or not, a cleaning liquid can be respectively introduced or discharged from the injection port and the exit port through the plastic hoses, so as to achieve cleaning the gene detection channel, thereby removing the non-hybridized gene, facilitating the observation of the hybridized gene, and improving the detection accuracy.

For example, in the abovementioned gene detection chip provided by the embodiment of the present disclosure, in order to be able to observe the reaction cell, the upper substrate is a transparent substrate.

In specific implementation, in the abovementioned gene detection chip provided by the embodiment of the present disclosure, the shape of the reaction cell can be a circle, or other shapes satisfying the design, which is not limited herein. For example, the shape of the reaction cell is designed as a circular shape, such that the reaction cell can be conveniently observed under a microscope.

In specific implementation, in the abovementioned gene detection chip provided by the embodiment of the present disclosure, the material of the lower substrate is glass. For example, in the abovementioned gene detection chip provided by the embodiment of the present disclosure, the material of the lower substrate is glass, which is used for silanization treatment, so that the aptamer having fluorescent light is bonded to the inner surface of the reaction cell and then the fluorescent light is quenched through graphite oxide. Thus, upon the sample solution with the target gene is hybridized with the aptamer on the surface of the reaction cell and then bonded to the surface of the reaction cell, the non-hybridized gene can be separated from the hybridized gene during the cleaning, thereby avoiding the influence of the non-hybridized gene on the hybridized gene in the detection result, which contributes to improving the accuracy of gene detection. In addition, the material of the upper substrate of the gene detection chip can be glass or a polymer such as polydimethyl siloxane. The injection port and exit port can be formed by forming through holes in the upper substrate. The microchannel and reaction cell can be formed by lithography on the lower substrate. In the reaction cell, the sample solution can perform hybridization reaction with the aptamer on the surface of the reaction cell. The upper and lower substrates can be bonded together with a low temperature adhesive.

For example, the gene detection chip provided by the embodiment of the present disclosure needs to quench the fluorescent light of the fluorescent label first upon performing the gene detection. Of course, in practical implementation, the fluorescent light of the fluorescent label can stay in a quenched state before the use of the gene detection chip.

Therefore, based on the same inventive concept, embodiments of the present disclosure further provide a gene detection chip, including an upper substrate and a lower substrate which are disposed opposite to each other.

The upper substrate is provided with at least one pair of ports including an injection port and an exit port; the injection port and the exit port can be respectively located at two opposite ends of the upper substrate.

A surface of the lower substrate facing the upper substrate is provided with at least one reaction cell and at least one microchannel, the reaction cell is respectively connected with the injection port and the exit port through the microchannel, wherein a surface of the reaction cell has a complex of an aptamer and a graphene oxide, the aptamer is modified with a fluorescent label, and the fluorescent light of the fluorescent label being in a quenched state.

In the gene detection chip provided by the embodiment of the present disclosure, compared with the abovementioned gene detection chip, the surface of the reaction cell of the former one has an aptamer modified with a fluorescent label, and the surface of the reaction cell in the present embodiment has a complex of an aptamer and a graphene oxide, the aptamer is modified with a fluorescent label and the fluorescent light of the fluorescent label being in a quenched state. In this way, since the fluorescent light is already quenched during use, it is not necessary to firstly quench the fluorescent light, but directly introducing the sample solution into the reaction cell through the injection. If the sample solution has the target gene, the target gene will hybridizes and bonds with the aptamer on the surface of the reaction cell, so as to recover the fluorescent light; if the sample solution does not have the target gene, the fluorescent light is still quenched. Therefore, the gene detection chip can detect the target gene without fluorescently labeling the target gene, which not only simplifies the process of gene detection, but also prevents the influence of the fluorescent signal of the non-hybridized target gene on the hybrid gene in the gene detection result. At the same time, the gene detection chip of the present disclosure has a simple structure, convenient operation, high specificity of aptamer, and accurate detection result.

Based on the same inventive concept, embodiments of the present disclosure provide a microfluidic control chip system, including: the abovementioned gene detection chip provided by the embodiments of the present disclosure and a power module; the power module is configured to supply power for introducing the sample solution into the gene detection chip and discharging the sample solution from the gene detection chip. Since the principle of the microfluidic control chip system for solving the problem is similar to that of the gene detection chip, the implementations of the microfluidic control chip system can refer to the implementations of the abovementioned gene detection chip, and the repeated portions will be omitted herein.

In specific implementation, in the microfluidic control chip system provided by the embodiment of the present disclosure, the power module can be a syringe pump. For example, in the abovementioned microfluidic control chip system provided by the embodiment of the present disclosure, a syringe pump can be used to supply power for introducing a liquid into the gene detection chip and discharging the liquid from the gene detection chip. Of course, other powers can also be used, which are not limited herein.

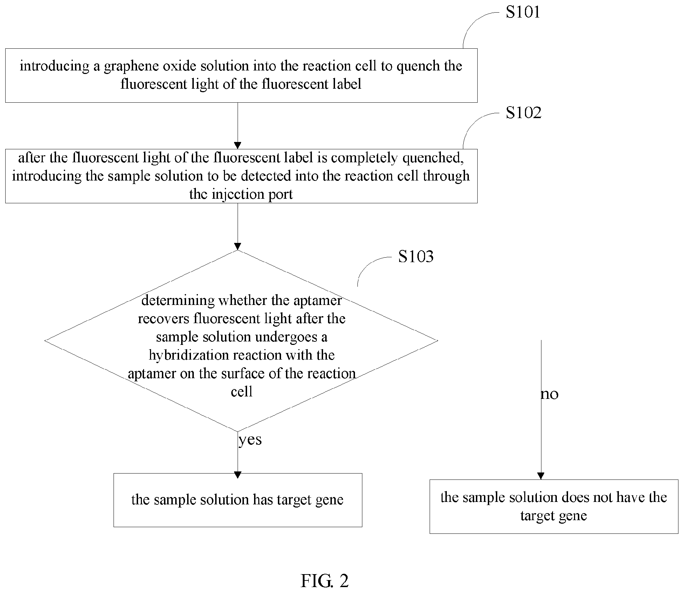

Based on the same inventive concept, embodiments of the present disclosure provide a detection method of the abovementioned gene detection chip provided by the embodiments of the present disclosure. As illustrated by Fig. 2, the detection method can include:
S101: introducing a graphene oxide solution into the reaction cell to quench the fluorescent light of the fluorescent label;
S102: after the fluorescent light of the fluorescent label is completely quenched, introducing the sample solution to be detected into the reaction cell through the injection port;
S103: determining whether the aptamer recovers fluorescent light after the sample solution undergoes a hybridization reaction with the aptamer on the surface of the reaction cell.

If the aptamer recovers fluorescent light, the sample solution has target gene, otherwise the sample solution does not have the target gene.

In the abovementioned detection method provided by the embodiment of the present disclosure, the target gene does not need to be labeled, but only introducing the graphene oxide solution into the reaction chamber to completely quench the fluorescent light of the fluorescent label, after the fluorescent light of the fluorescent label is completely quenched, introducing the sample solution to be detected into the reaction cell, such that the sample solution to be detected and the aptamer located on the surface of the reaction cell and modified with fluorescent label whose fluorescent light is completely quenched undergo a hybridization reaction in the reaction cell, thereby achieve detecting the target gene. The gene detection method is convenient in operation and the detection result is obtained with high accuracy.

For example, graphene oxide is a derivative of graphene, which incorporates a hydroxyl group and a carboxyl group on the basis of graphene; compared with graphene, the graphene oxide has stronger hydrophilicity and bio compatibility. As a quencher, the graphene oxide can quench the fluorescent light of most organic dyes and quantum dots. The graphene oxide can adsorb single-stranded nucleic acid aptamer, and then quench the fluorescent light of the label. Therefore, graphene oxide is added into the reaction cell to mix with the aptamer modified with a fluorescent label, so as to achieve quenching. Upon the aptamer being combined with the complementary DNA in the target gene to form a double-stranded DNA molecule; as the steric hindrance becomes larger, the adsorption ability of the graphene oxide is weakened, and the fluorescent light recovers, so as to achieve detecting the target gene. Therefore, during the detection, the sample solution is introduced from the injection port, and reacts with the aptamer on the surface of the reaction cell. Upon the sample solution to be detected having the target gene, the gene and the aptamer are combined and detached from the graphene oxide, and a corresponding florescent light signal can be obtained; otherwise, upon the sample solution to be detected not having the target gene, all of the fluorescent light stays in a quenched state, and there is no fluorescent light signal. To sum up, the gene detection method provided by the embodiment of the present disclosure removes the complicated labeling process of the target gene, simplifies the gene detection technology, combines the microfluidic chip technology with gene detection, and, uses the microfluidic technology to continuously control micro-fluid on a micron-scale chip to achieve gene detection. The gene detection method has the features that the reagent consumption is small, the volume is portable, and the reaction is fast, efficient, and easy to integrate.

In specific implementation, in the abovementioned detection method provided by the embodiment of the present disclosure, before determining whether the aptamer recovers fluorescent light after the sample solution undergoes a hybridization reaction with the aptamer on the surface of the reaction cell, the detection method may further include: cleaning the reaction cell.

For example, non-hybridized fluorescent probes can be washed away by the cleaning process, so as to prevent interference. The specific cleaning process includes: introducing a cleaning liquid into the reaction cell through the injection port, and discharging the cleaning liquid out through the exit port.

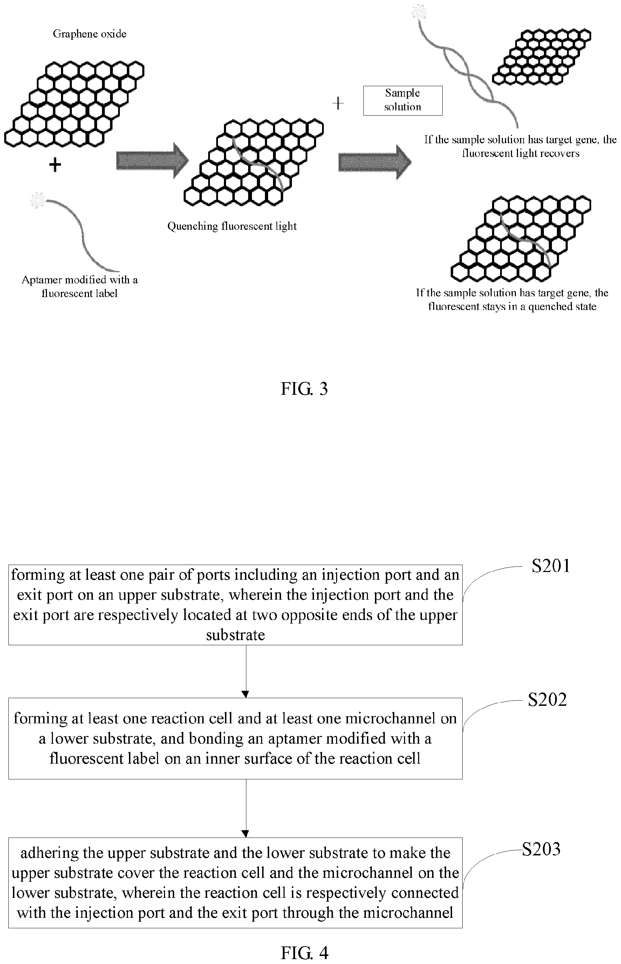

Based on the same inventive concept, embodiments of the present disclosure provide a manufacturing method of a gene detection chip, as illustrated by Fig. 4, the manufacturing method includes:
S201: forming at least one pair of ports including an injection port and an exit port on an upper substrate, wherein the injection port and the exit port are respectively located at two opposite ends of the upper substrate;
S202: forming at least one reaction cell and at least one microchannel on a lower substrate, and bonding an aptamer modified with a fluorescent label on an inner surface of the reaction cell;
S203: adhering the upper substrate and the lower substrate to make the upper substrate cover the reaction cell and the microchannel on the lower substrate, wherein the reaction cell is respectively connected with the injection port and the exit port through the microchannel.

For example, in the abovementioned manufacturing method provided by the embodiment of the present disclosure, step S201 and step S202 may be performed without sequence, that is, step S201 can be performed first, and then step S202 can be performed; or, step S202 can be performed first and then step S201 can be performed. Certainly, Step S201 and step S202 can be performed simultaneously, which is not limited herein.

For example, in the abovementioned manufacturing method provided by the embodiment of the present disclosure, bonding an aptamer modified with a fluorescent label on an inner surface of the reaction cell includes:
performing a silanization treatment on the inner surface of the reaction cell, wherein a material of the lower substrate is glass;
using an array machine to bond the aptamer modified with the fluorescent label on the inner surface of the reaction cell.

For example, after the silanization treatment is performed, the aptamer modified with a fluorescent label can be bonded to the inner surface of the reaction cell, so that after the hybridization reaction, the sample solution with the target gene hybridizes with the aptamer and then is bonded on the surface of the reaction cell. The non-hybridized gene and hybridized gene can be separated during the cleaning process, thereby avoiding the influence of the non-hybridized gene on the hybridized gene, and contributing to improving the accuracy of the gene detection.

For example, in order to avoid the need to completely quench the fluorescent light of the fluorescent label upon performing the gene detection, in the abovementioned manufacturing method provided in the embodiment of the present disclosure, after bonding an aptamer modified with a fluorescent label on an inner surface of the reaction cell of the step S202, the manufacturing method further includes::
introducing a graphene oxide solution into the reaction cell to completely quench fluorescent light of the fluorescent label;
cleaning the reaction cell.

For example, in the abovementioned manufacturing method provided by the embodiment of the present disclosure, the graphene oxide solution can be firstly introduced into the reaction cell, and then the upper substrate and the lower substrate are adhered; certainly, the graphene oxide solution can be introduced into the reaction cell through the injection port after adhering the upper substrate and the lower substrate, which is not limited herein.

For example, in the abovementioned manufacturing method provided by the embodiment of the present disclosure, the upper substrate and the lower substrate are generally adhered by a low-temperature adhesive.

For example, in the abovementioned manufacturing method provided by the embodiment of the present disclosure, an injection port and an exit port can be formed on the upper substrate by forming through holes.

For example, in the abovementioned manufacturing method provided by the embodiment of the present disclosure, the microchannel and the reaction cell can be formed by etching the lower substrate through photolithography technology.

The detection principle of the abovementioned gene detection chip provided by the embodiments of the present disclosure will be described in detail by a specific embodiment as follows.

As illustrated by Fig. 3, the reaction cell of the gene detection chip is bonded with an aptamer modified with a fluorescent dye through a chemical surface treatment; then 1 mg/ml of graphene oxide solution was introduced into the reaction cell. A fluorescent light confocal microscope can be used to observe the quenching situation until the fluorescent light is completely quenched. In a case where a sample solution to be detected is added into the reaction cell of the chip, if the sample solution has the target gene, the target gene will bond with the aptamer on the surface of the glass, so that the fluorescent light recovers; if the sample solution does not have the target gene, the aptamer cannot be combined and remain a quenched state.

The abovementioned gene detection chip provided by the embodiment of the present disclosure can achieve the purpose of gene detection, the specificity of the aptamer is high and the detection result is accurate. At the same time, the disclosed gene detection chip can have a plurality of parallel channels. For example, as illustrated by Fig. 1, there are four parallel channels, which can simultaneously detect four different genes, and the detection efficiency is fast and efficient

The embodiments of the present disclosure provide a gene detection chip, a detection method thereof, a manufacturing method thereof, and a micro fluidic control chip system. The gene detection chip includes: an upper substrate and a lower substrate which are disposed opposite to each other; wherein the upper substrate is provided with at least one pair of ports including an injection port and an exit port, the injection port and the exit port are respectively located at two opposite ends of the upper substrate; a surface of the lower substrate facing the upper substrate is provided with at least one reaction cell and at least one microchannel; and the reaction cell is respectively connected with the injection port and the exit port through the microchannel; a gene detection channel is formed by an injection port, a microchannel, a reaction cell, and an exit port. Since the surface of the reaction cell has an aptamer, and the aptamer is modified with a fluorescent label, upon a gene detection being performed, firstly quenching the fluorescent light of the fluorescent label, and then introducing a sample solution into the reaction cell through the injection port; if the sample solution has target gene, the target gene will hybridize and combine with the aptamer on the surface of the reaction cell, so as to recover the fluorescent light; if the sample solution does not have the target gene, the fluorescent light stays in the quenched state. Therefore, the gene detection chip can achieve detecting the target gene without fluorescently labeling the target gene, which not only simplifies the process of gene detection, but also prevents the influence of the fluorescent signal of the non-hybridized target gene on the hybrid gene in the gene detection result. At the same time, the gene detection chip provided by the present disclosure has a simple structure, convenient operation, high specificity of aptamer, and accurate detection result.

Apparently, those skilled in the art can make various modifications and variations to the embodiments of the present disclosure without departing from the spirit and scope of the embodiments of the disclosure. Thus, if these modifications and variations to the embodiments of the present disclosure fall within the scope of the claims of the present application and their equivalent technologies, the present application is also intended to include these modifications and variations.

## Claims

1. A gene detection chip, comprising: an upper substrate and a lower substrate which are disposed opposite to each other;
wherein the upper substrate is provided with at least one pair of ports comprising an injection port and an exit port, the injection port and the exit port are respectively located at two opposite ends of the upper substrate;
a surface of the lower substrate facing the upper substrate is provided with at least one reaction cell and at least one microchannel, the reaction cell is respectively connected with the injection port and the exit port through the microchannel, wherein a surface of the reaction cell has an aptamer, the aptamer is modified with a fluorescent label.

2. The gene detection chip according to claim 1, further comprising: a first plastic hose connected to the injection port and a second plastic hose connected to the exit port;
wherein the first plastic hose is configured to introduce a solution through the injection port;
the second plastic hose is configured to discharge the solution through the exit port.

3. The gene detection chip according to claim 1 or 2, wherein a shape of the reaction cell comprises a circular shape.

4. The gene detection chip according to any one of claims 1-3, wherein a material of the lower substrate comprises glass.

5. The gene detection chip according to any one of claims 1-4, wherein the upper substrate is a transparent substrate.

6. A microfluidic control chip system, comprising: the gene detection chip according to any one of claims 1-5 and a power module;
wherein the power module is configured to supply power for introducing a solution into the gene detection chip or discharging the solution from the gene detection chip.

7. The microfluidic control chip system of claim 6, wherein the power module is a syringe pump.

8. A detection method of the gene detection chip according to any one of claims 1-5, comprising:
introducing a graphene oxide solution into the reaction cell to completely quench fluorescent light of the fluorescent label;
after completely quenching fluorescent light of the fluorescent label, introducing a sample solution to be detected into the reaction cell through the injection port; and
determining whether the aptamer recovers fluorescent light after the sample solution undergoes a hybridization reaction with the aptamer on the surface of the reaction cell, and if the aptamer recovers the fluorescent light, the sample solution has a target gene, otherwise the sample solution does not have the target gene.

9. The detection method according to claim 8, wherein before determining whether the aptamer recovers the fluorescent light after the sample solution undergoes the hybridization reaction with the aptamer on the surface of the reaction cell, the detection method further comprises:
cleaning the reaction cell.

10. The detection method according to claim 9, wherein cleaning the reaction cell comprises:
introducing a cleaning liquid into the reaction cell through the injection port, and discharging the cleaning liquid from the exit port.

11. A manufacturing method of a gene detection chip, comprising:
forming at least one pair of ports comprising an injection port and an exit port on an upper substrate, wherein the injection port and the exit port are respectively located at two opposite ends of the upper substrate;
forming at least one reaction cell and at least one microchannel on a lower substrate, and bonding an aptamer modified with a fluorescent label on an inner surface of the reaction cell; and
adhering the upper substrate and the lower substrate to make the upper substrate cover the reaction cell and the microchannel on the lower substrate, wherein the reaction cell is respectively connected with the injection port and the exit port through the microchannel.

12. The manufacturing method according to claim 11, wherein bonding the aptamer modified with the fluorescent label on the inner surface of the reaction cell comprises:
performing a silanization treatment on the inner surface of the reaction cell, wherein a material of the lower substrate is glass; and
using an array machine to bond the aptamer modified with the fluorescent label on the inner surface of the reaction cell.

13. The manufacturing method according to claim 11, wherein after bonding the aptamer modified with the fluorescent label on the inner surface of the reaction cell, the manufacturing method further comprises:
introducing a graphene oxide solution into the reaction cell to completely quench fluorescent light of the fluorescent label; and
cleaning the reaction cell.

14. A gene detection chip, comprising: an upper substrate and a lower substrate which are disposed opposite to each other;
wherein the upper substrate is provided with at least one pair of ports comprising an injection port and an exit port, the injection port and the exit port are respectively located at two opposite ends of the upper substrate;
a surface of the lower substrate facing the upper substrate is provided with at least one reaction cell and at least one microchannel, the reaction cell is respectively connected with the injection port and the exit port through the microchannel, wherein a surface of the reaction cell has a complex of an aptamer and a graphene oxide, the aptamer is modified with a fluorescent label, and fluorescent light of the fluorescent label being in a quenched state.
